# EUROPEAN PATENT APPLICATION

(11) **EP 3 904 535 A1**
(43) Date of publication of application: **03.11.2021**
(21) Application number: 19905657.3
(22) Date of filing: 24.12.2019
(51) Int. Cl.: C12Q 1/6886, C12Q 1/6883

(54) **HEALTH RISK ASSESSMENT METHOD**

(30) Priority: 24.12.2018 US 201862784541 P
(71) Applicant: Quark Biosciences Taiwan, Inc., Zhubei City, Hsinchu County 30261 (TW)
(72) Inventor: CHEN, Wei Ming, Zhubei City, Hsinchu County Taiwan 30261 (TW); KANG, Shih Ting, Zhubei City, Hsinchu County Taiwan 30261 (TW)
(74) Representative: Becker, Eberhard
(86) International application number: PCT/CN2019/127934
(87) International publication number: WO 2020/135422

(57) **Abstract**

The disclosure provides a health risk assessment method including the following steps. A miRNA expression database of a healthy population is established. A miRNA expression in a plasma specimen of a subject is analyzed. After that, miRNA expression data of the subject is compared with the miRNA expression in the miRNA expression database of the healthy population, and the miRNA expression being too high or too low in the plasma specimen of the subject is found out to assess health risk of the subject.

## Description

### BACKGROUND

### Technical Field

The disclosure relates to a health risk assessment method, and more particularly to a health risk assessment method through microRNA (miRNA) expression analysis.

### Description of Related Art

A microRNA (miRNA) is a non-coding RNA with a length of approximately 18 to 25 nucleotides that has been highly preserved during the evolution process and plays a very important role in intracellular regulation. In 1993, the miRNA was first discovered in *Caenorhabditis elegans (C. elegans).* One after another, more and more miRNAs have been discovered in humans and other species. At present, there are approximately 2,500 known miRNAs in human cells, and these miRNAs have been proven to regulate more than 50% messenger RNAs' (mRNA) expression. In addition, abnormal miRNA expression has been proven to be closely related to formation of many diseases, including cancer diseases, chronic diseases, and autoimmune diseases.

During the past few years, the miRNA has been widely praised and regarded as a new molecular detection target. At present, the miRNA has also been proven to be secreted from cells into blood, forming protein-RNA complexes to ensure that the miRNA will not be degraded by ribonuclease (RNase). Such feature has become very valuable for making free miRNAs in the blood relatively easy to obtain and allowing cell-free miRNA expression profiling to be a basis for initial diagnosis of diseases. For example, different types of cancer have been proven to have unique miRNA expression profiles, or miRNA signatures, which may be used as a basis for initial diagnosis of cancer.

For a long time, developing non-invasive disease detection methods with convenience and high diagnostic rates has been a goal consistently pursued by the medical community. Take cancer as an example, cancer screening may be carried out to find potential, undetected initial-stage asymptomatic cancer early. The cancer screening refers to a process of using examinations, tests, or other methods to identify whether a patient has cancer.

At present, patients may be diagnosed for cancer through many symptoms or test results, but the most certain way to diagnose malignant tumors is still an invasive detection method that a pathologist conducts a biopsy of living tissues or performs a pathological test on tissues obtained by surgery to confirm the presence of cancer cells.

In addition, tumor marker detection refers to determination of cancer by detecting changes in special proteins associated with malignant tumor cells. However, sensitivity and specificity of the tumor marker detection is not ideal, and cancer cells are often detected as late as when the tumor has developed to a considerable size or has metastasized to other organs.

Based on the above, developing a non-invasive, early health risk assessment method for immediate, real-time monitoring of health risk from diseases is an important topic for current research.

### SUMMARY

The disclosure provides a health risk assessment method which monitors and assesses health risk in real time through microRNA (miRNA) expression analysis.

The health risk assessment method of the disclosure includes the following steps. First, a miRNA expression database of a healthy population is established, and a miRNA expression of a plasma specimen of a subject is analyzed. After that, miRNA expression data of the subject is compared with the miRNA expression in the miRNA expression database of the healthy population, and the miRNA expression being too high or too low in the plasma specimen of the subject is found out to assess health risk of the subject.

In an embodiment of the disclosure, determination of the health risk includes cancer or diabetes.

In an embodiment of the disclosure, the miRNAs are categorized into an H type, an M type, an L type, or a Cn type based on their miRNA expression for the miRNA expression database of the healthy population. The H type represents a detection frequency of its miRNA expression in the healthy population is higher than 60%. The M type represents the detection frequency of its miRNA expression in the healthy population is 20% to 60%. The L type represents the detection frequency of its miRNA expression in the healthy population is lower than 20%. The Cn type represents no miRNA expression is detected in this type in the healthy population.

In an embodiment of the disclosure, the miRNAs are categorized into a U type, a D type, an N type, or an En type based on their miRNA expression for the miRNA expression data of the subject. The U type represents its miRNA expression of the subject is higher than its miRNA expression reference interval of the healthy population. The D type represents its miRNA expression of the subject is lower than its miRNA expression reference interval of the healthy population. The N type represents its miRNA expression of the subject falls in its miRNA expression reference interval of the healthy population. The En type represents no miRNA expression of the subject is detected.

In an embodiment of the disclosure, each miRNA type of the subject is categorized into a first population, a second population, a third population, a fourth population, or a fifth population. A red dot represents each miRNA type belongs to the first population, the second population, the third population, the fourth population, or the fifth population. The first population represents its miRNAs belong to both the H type and the U type. The second population represents its miRNAs belong to both the M type and the U type. The third population represents its miRNAs belong to both the Cn type and the U type. The fourth population represents its miRNAs belong to both the H type and the D type. The fifth population represents its miRNAs belong to both the M type and the D type.

In an embodiment of the disclosure, a number of the red dots higher than or equal to 5 represents the subject has the health risk.

Based on the above, a non-invasive, early health risk assessment method is provided, analyzing the miRNA expression in the plasma of the subject for comparison with the miRNA expression database of the healthy population. Therefore, the health risk assessment may assess the health risk immediately and efficiently, and may further improve convenience and diagnostic rates of existing health risk screening.

In order to make the aforementioned features and advantages of the disclosure comprehensible, embodiments are described in detail below.

### DESCRIPTION OF THE EMBODIMENTS

The disclosure provides an improved nucleic acid specimen measurement method including the following steps. First, a miRNA expression database of a healthy population is established, and a miRNA expression in a plasma specimen of a subject is analyzed. After that, miRNA expression data of the subject is compared with the miRNA expression in the miRNA expression database of the healthy population, and the miRNA expression being too high or too low in the plasma specimen of the subject is found out to assess health risk of the subject.

In this embodiment, a method of establishing the miRNA expression database of the healthy population is as follows. First, a miRNA information database related to diseases is established based on more than 30,000 articles, and 167 miRNAs highly related to the diseases are screened out. More than 300 healthy subjects that have not been diagnosed by a physician to have cancer/diabetes/or other major diseases are enrolled. After the physician assesses that the subjects have no tumor risk, plasma specimens are collected to detect 167 miRNA expression profiles in the plasma specimens for calculating an average and a standard deviation of each miRNA expression in the healthy population. Accordingly, a normal range of each miRNA expression in the healthy population is calculated to establish a database for the 167 miRNA expression profiles in the healthy population.

In this embodiment, the 167 miRNAs screened out to be highly related to the diseases are shown in Table 1 below.

**Table 1**

| 167 miRNA biomarkers | | | | |
|---|---|---|---|---|
| hsa-let-7d-3p | hsa-miR-214-3p | hsa-let-7b-5p | hsa-miR-20b-5p | hsa-miR-451a |
| hsa-miR-101-3p | hsa-miR-215-5p | hsa-let-7c-5p | hsa-miR-210-3p | hsa-miR-23a-3p |
| hsa-miR-122-5p | hsa-miR-21-5p | hsa-let-7d-5p | hsa-miR-217 | hsa-let-7a-5p |
| hsa-miR-1254 | hsa-miR-216a-5p | hsa-let-7f-5p | hsa-miR-221-3p | hsa-miR-103a-3p |
| hsa-miR-125b-5p | hsa-miR-222-3p | hsa-let-7g-5p | hsa-miR-223-3p | hsa-miR-16-5p |
| hsa-miR-126-3p | hsa-miR-22-3p | hsa-miR-100-5p | hsa-miR-27a-3p | hsa-miR-191-5p |
| hsa-miR-1290 | hsa-miR-224-5p | hsa-miR-106a-5p | hsa-miR-29a-3p | hsa-miR-423-3p |
| hsa-miR-129-5p | hsa-miR-24-3p | hsa-miR-106b-5p | hsa-miR-29b-3p | hsa-miR-423-5p |
| hsa-miR-140-5p | hsa-miR-26a-5p | hsa-miR-10a-5p | hsa-miR-29c-3p | hsa-miR-93-5p |
| hsa-miR-142-3p | hsa-miR-28-3p | hsa-miR-10b-5p | hsa-miR-302d-3p | hsa-miR-425-5p |
| hsa-miR-143-3p | hsa-miR-299-5p | hsa-miR-1248 | hsa-miR-30a-5p | hsa-miR-1228-5p |
| hsa-miR-144-3p | hsa-miR-29a-5p | hsa-miR-125a-5p | hsa-miR-30c-5p | |
| hsa-miR-145-5p | hsa-miR-30b-5p | hsa-miR-127-3p | hsa-miR-30d-5p | |
| hsa-miR-146a-5p | hsa-miR-31-5p | hsa-miR-128-3p | hsa-miR-30e-5p | |
| hsa-miR-150-5p | hsa-miR-326 | hsa-miR-130a-3p | hsa-miR-31-3p | |
| hsa-miR-151a-3p | hsa-miR-335-5p | hsa-miR-130b-3p | hsa-miR-320a | |
| hsa-miR-152-3p | hsa-miR-338-5p | hsa-miR-133a-3p | hsa-miR-330-5p | |
| hsa-miR-155-5p | hsa-miR-34a-5p | hsa-miR-133b | hsa-miR-376c-3p | |
| hsa-miR-15a-5p | hsa-miR-361-5p | hsa-miR-134-5p | hsa-miR-411-5p | |
| hsa-miR-15b-5p | hsa-miR-372-3p | hsa-miR-135a-5p | hsa-miR-429 | |
| hsa-miR-17-3p | hsa-miR-373-3p | hsa-miR-135b-5p | hsa-miR-4306 | |
| hsa-miR-181a-5p | hsa-miR-375 | hsa-miR-1-3p | hsa-miR-450a-5p | |
| hsa-miR-181b-5p | hsa-miR-378a-5p | hsa-miR-141-3p | hsa-miR-486-5p | |
| hsa-miR-182-5p | hsa-miR-382-5p | hsa-miR-146b-5p | hsa-miR-518a-5p | |
| hsa-miR-183-5p | hsa-miR-409-3p | hsa-miR-17-5p | hsa-miR-520b | |
| hsa-miR-193a-3p | hsa-miR-425-3p | hsa-miR-18a-5p | hsa-miR-539-5p | |
| hsa-miR-1972 | hsa-miR-452-3p | hsa-miR-18b-5p | hsa-miR-625-5p | |
| hsa-miR-197-3p | hsa-miR-483-5p | hsa-miR-192-5p | hsa-miR-652-3p | |
| hsa-miR-198 | hsa-miR-484 | hsa-miR-193b-3p | hsa-miR-660-5p | |
| hsa-miR-199a-5p | hsa-miR-499a-5p | hsa-miR-195-5p | hsa-miR-663a | |
| hsa-miR-19a-3p | hsa-miR-500a-5p | hsa-miR-196a-5p | hsa-miR-718 | |
| hsa-miR-19b-3p | hsa-miR-574-3p | hsa-miR-196b-5p | hsa-miR-7-5p | |
| hsa-miR-202-3p | hsa-miR-574-5p | hsa-miR-1973 | hsa-miR-760 | |
| hsa-miR-203a-3p | hsa-miR-579-3p | hsa-miR-199a-3p | hsa-miR-885-5p | |
| hsa-miR-205-5p | hsa-miR-589-5p | hsa-miR-200a-3p | hsa-miR-92a-3p | |
| hsa-miR-206 | hsa-miR-593-5p | hsa-miR-200b-3p | hsa-miR-95-3p | |
| hsa-miR-20a-5p | hsa-miR-596 | hsa-miR-200c-3p | hsa-miR-9-5p | |
| hsa-miR-2114-3p | hsa-miR-601 | hsa-miR-208b-3p | hsa-miR-99b-5p | |
| hsa-miR-940 | hsa-miR-1225-3p | hsa-miR-223-5p | hsa-miR-4772-3p | |

In this embodiment, a method of detecting the miRNAs in plasma includes the following steps.

### 1. Collecting blood specimens

A puncture site on skin of a blood sampling target is wiped with alcohol, and a tourniquet is tied 5 cm to 15 cm above the puncture site with a slip knot. 10 ml of whole blood is drawn up with a 19G to 22G needle into a K₂EDTA BD Vacutainer blood collection tube, and the tourniquet should be released immediately after the blood flows into the blood collection tube. After blood drawing, the blood collection tube is immediately turned upside down 5 to 8 times for mixing to ensure that an anticoagulant has fully functioned. The blood collection tube is stored at room temperature, and a plasma separation step should be completed within one hour after blood collection.

### 2. Plasma separation method

The blood collection tube is placed on a swinging-bucket rotor and centrifuged at 1,200 xg for 10 minutes at room temperature. After centrifugation, supernatant is taken out to a 15 ml centrifuge tube. The supernatant in the 15 ml centrifuge tube is drawn up and released 5 times with a pipette to ensure mixing, and is evenly divided into 1.5 ml DNase/RNase-free eppendorf tubes for centrifugation at 12,000 xg for 10 minutes at room temperature. After centrifugation, the supernatant is taken out to a new 15 ml centrifuge tube, without taking white sediment at the bottom of the 1.5 ml eppendorf tubes. The supernatant is drawn up and released 5 times with the pipette to ensure mixing, and is evenly divided into 1.5 ml DNA LoBind Tubes (Eppendorf, 22431021) and immediately stored in a refrigerator at -80 degrees Celsius.

### 3. miRNA extraction method

The plasma specimen is taken out from the refrigerator at -80 degrees Celsius to thaw on ice, and an experiment is performed according to an operations manual provided by Qiagen miRNeasy Serum/Plasma Kit after thawing for reconstitution with 30 µl nuclease-free water.

### 4. cDNA synthesis

Appropriate quantities of miRNAs are taken for performing a reverse transcription reaction with a Quarkbio miRNA Universal RT kit to synthesize cDNAs.

### 5. qPCR experiment

Appropriate quantities of cDNAs are taken for performing a qPCR experiment with an operations manual provided by Quarkbio miRSCAN Panelchip^{®}.

In this embodiment, determination of the health risk may include, but not limited to, cancer or diabetes, and may also include other diseases or risk factors that may have adverse effects on health.

In this embodiment, the miRNAs are categorized into an H type, an M type, an L type, or a Cn type based on their miRNA expression for the miRNA expression database of the healthy population. The H type represents a detection frequency of its miRNA expression in the healthy population is higher than 60%. The M type represents the detection frequency of its miRNA expression in the healthy population is 20% to 60%. The L type represents the detection frequency of its miRNA expression in the healthy population is lower than 20%. The Cn type represents no miRNA expression is detected in this type in the healthy population.

In an embodiment of the disclosure, the miRNAs are categorized into a U type, a D type, an N type, or an En type based on their miRNA expression for the miRNA expression data of the subject. The U type represents its miRNA expression of the subject are higher than its miRNA expression reference interval of the healthy population. The D type represents its miRNA expression of the subject are lower than its miRNA expression reference interval of the healthy population. The N type represents its miRNA expression of the subject falls in its miRNA expression reference interval of the healthy population. The En type represents no miRNA expression of the subject is detected in this type.

In this embodiment, each miRNA type of the subject is categorized into a first population, a second population, a third population, a fourth population, or a fifth population. A red dot represents each miRNA type belongs to the first population, the second population, the third population, the fourth population, or the fifth population. The first population represents its miRNAs belong to both the H type and the U type. The second population represents its miRNAs belong to both the M type and the U type. The third population represents its miRNAs belong to both the Cn type and the U type. The fourth population represents its miRNAs belong to both the H type and the D type. The fifth population represents its miRNAs belong to both the M type and the D type. A number of the red dots higher than or equal to 5 represents the subject has the health risk.

Hereinafter, the health risk assessment method of the aforementioned embodiment will be described in detail through experimental examples. However, the following experimental examples are not intended to limit the disclosure.

### Experimental Examples

In order to prove that the health risk assessment method proposed by the disclosure may assess health risk immediately and efficiently, an experimental example is particularly made as follows.

### Example 1: Cancer risk assessment

198 subjects known to be in a low-risk population and a cancer population (as determined by a physician) were analyzed, and the number of red dots of each subject was determined through the determination method mentioned in the above embodiment to obtain results shown in Table 2 below. In Table 2, the low-risk population refers to subjects who have not been diagnosed with any major diseases, and the cancer population refers to subjects who has not been treated. Among 198 test specimens, accuracy rate of successfully identifying a high-risk population having potential health risk was about 73%.

**Table 2**

| Sample Population | Number of Specimens | Red Dot Statistical Range | | | | Number of Specimens with Red Points ≥ 5 |
|---|---|---|---|---|---|---|
| | | first quartile | median | third quartile | average | |
| low-risk population | 25 | 2 | 3 | 5 | 3.32 | 8 |
| early breast cancer population | 30 | 3.25 | 5 | 6 | 5.03 | 16 |
| oral cancer population | 57 | 4 | 6 | 7 | 6.07 | 37 |
| pan-cancer population | 86 | 7 | 11 | 16 | 12.4 | 74 |

### Example 2: Diabetes risk assessment

56 subjects known to be in a low-risk population and a diabetes population (as determined by a physician) were analyzed, and the number of red dots of each subject was determined through the determination method mentioned in the above embodiment to obtain results shown in Table 3 below. In Table 3, the low-risk population refers to subjects who have not been diagnosed with any major diseases. Among 56 test specimens, accuracy rate of successfully identifying a high-risk population having potential health risk was about 70%.

**Table 3**

| Sample Population | Number of Specimens | Red Dot Statistical Range | | | | Number of Specimens with Red Points ≥ 5 |
|---|---|---|---|---|---|---|
| | | first quartile | median | third quartile | average | |
| low-risk population | 25 | 2 | 3 | 5 | 3.32 | 8 |
| diabetes population | 31 | 4 | 6 | 9 | 6.55 | 22 |

In summary, the disclosure provides a non-invasive, early health risk assessment method which analyzes the miRNA expression in the plasma of the subject for comparison with the miRNA database of the healthy population. Therefore, the health risk assessment may assess the health risk immediately and efficiently, and may further improve convenience and diagnostic rates of existing health risk screening, providing personalized professional health risk monitoring.

Although the disclosure has been described with reference to the above embodiments, it will be apparent to one of ordinary skill in the art that modifications to the described embodiments may be made without departing from the spirit of the disclosure. Accordingly, the scope of the disclosure will be defined by the attached claims and their equivalents and not by the above detailed descriptions.

## Claims

1. A health risk assessment method, comprising:
establishing a miRNA expression database of a healthy population;
analyzing a miRNA expression in a plasma specimen of a subject; and
comparing miRNA expression data of the subject with the miRNA expression in the miRNA expression database of the healthy population and finding out the miRNA expression being too high or too low in the plasma specimen of the subject to assess health risk of the subj ect.

2. The health risk assessment method according to claim 1, wherein determination of the health risk comprises cancer or diabetes.

3. The health risk assessment method according to claim 1, wherein miRNAs are categorized into an H type, an M type, an L type, or a Cn type based on a detection frequency of each miRNA expression for the miRNA expression database of the healthy population, wherein the H type represents the detection frequency is higher than 60%, the M type represents the detection frequency is 20% to 60%, the L type represents the detection frequency is lower than 20%, and the Cn type represents no miRNA expression is detected in this type.

4. The health risk assessment method according to claim 3, wherein the miRNAs are categorized into a U type, a D type, an N type, or an En type based on the miRNA expression for the miRNA expression data of the subject, wherein the U type represents its miRNA expression of the subject is higher than its miRNA expression reference interval of the healthy population, the D type represents its miRNA expression of the subject is lower than its miRNA expression reference interval of the healthy population, the N type represents its miRNA expression of the subject falls in its miRNA expression reference interval of the healthy population, and the En type represents no miRNA expression of the subject is detected in this type.

5. The health risk assessment method according to claim 4, wherein each miRNA type of the subject is categorized into a first population, a second population, a third population, a fourth population, or a fifth population, and a red dot represents each miRNA type belongs to the first population, the second population, the third population, the fourth population, or the fifth population, wherein the first population represents its miRNAs belong to both the H type and the U type, the second population represents its miRNAs belong to both the M type and the U type, the third population represents its miRNAs belong to both the Cn type and the U type, the fourth population represents its miRNAs belong to both the H type and the D type, and the fifth population represents its miRNAs belong to both the M type and the D type.

6. The health risk assessment method according to claim 5, wherein a number of the red dots higher than or equal to 5 represents the subject has the health risk.
